Europäisches Patentamt

European Patent Office

Office européen des brevets

(11) Veröffentlichungsnummer : **0 123 029**
**B1**

(12) EUROPÄISCHE PATENTSCHRIFT

(45) Veröffentlichungstag der Patentschrift :
29.07.87

(21) Anmeldenummer : 84101219.8

(22) Anmeldetag : 07.02.84

(51) Int. Cl.⁴ : **A 61 K 39/395, C 07 K 3/28,**
**C 07 K 15/06**

(54) Verfahren zur Herstellung einer nebenwirkungsfreien IgG-Immunglobulinlösung für die intravenöse Applikation.

(30) Priorität : 21.03.83 DE 3310150

(43) Veröffentlichungstag der Anmeldung :
31.10.84 Patentblatt 84/44

(45) Bekanntmachung des Hinweises auf die Patenterteilung : 29.07.87 Patentblatt 87/31

(84) Benannte Vertragsstaaten :
AT BE CH DE FR GB IT LI LU NL SE

(56) Entgegenhaltungen :
DE-A- 2 404 265

(73) Patentinhaber : SCHWAB & CO. GES.M.B.H.
Doerenkampgasse 4
A-1100 Wien (AT)
BE CH FR GB IT LI LU NL SE AT
Lentia Gesellschaft mit beschränkter Haftung
Arabellastrasse 4 Postfach 81 05 08
D-8000 München 81 (DE)
DE

(72) Erfinder : Wahl, Heinrich
Am Waldrand 10
A-2500 Sooss (AT)
Erfinder : Frolik, Manfred
Kierlingergasse 19
A-3420 Kritzendorf (AT)
Erfinder : Doleschel, Walter, Prof. Dr.
Sonnleitensteig 7
A-1190 Wien (AT)

(74) Vertreter : Kunz, Ekkehard, Dr.
Chemie Linz AG Patentabteilung St. Peter-Strasse 25
A-4020 Linz (AT)

## Beschreibung

Die vorliegende Erfindung betrifft ein verbessertes Verfahren für die Herstellung einer chemisch unmodifizierten, nebenwirkungsfreien und weitgehend reinen IgG-Immunglobulinfraktion mit gegenüber natürlichem Plasma unveränderter Komplement bindender Aktivität, die in Form einer stabilen, klaren wässerigen Lösung für die intravenöse Injektion zur Verfügung steht.

Die übliche Klassifizierung der Immunglobuline erfolgt nach Bull. World Health Org. 30, 1964, 447 durch Bezeichnung mit Symbolen, die sowohl die Hauptklasse als auch die in diesen Globulinen vorkommenden Polypeptidketten angeben. Dementsprechend wird erfindungsgemäß das Zeichen Ig für Immunglobuline benutzt, wobei ein Großbuchstabe zur näheren Bezeichnung der jeweiligen Immunglobulinklasse nachgestellt wird. Monomeres IgG-Immunglobulin wird der Literatur entsprechend als 7S IgG bezeichnet.

Die mit IgG bezeichnete Immunglobulinfraktion aus gepooltem Plasma enthält Antikörper gegen verschiedene pathogene Mikroorganismen und wird u. a. zur Prophylaxe und Therapie von Virusinfektionen und bakteriellen Infektionen eingesetzt. Daher ist die Isolierung und Reinigung von IgG-Immunglobulin für die Bereitstellung von passiven Immunisierungspräparaten in der Medizin besonders wichtig.

Es sind bereits verschiedene Verfahren zur Gewinnung von Immunglobulinen aus menschlichem Plasma oder Serum bekannt. Beispielsweise kann IgG-Immunglobulin mittels des Alkoholfällungsverfahrens von Cohn et al. (J. Am. Chem. Soc. 68, 459-475, 1946 und ibid. 72, 465-474, 1950, weiterentwickelt in US-PS-3 597 409) in den Plasmafraktionen II oder II + III angereichert werden. Eine andere bekannte Methode besteht in der Kombination von Fällungsschritten mit Rivanol und Ammoniumsulfat (J. Horejisi, R. Smetana ; Acta Med. Scand. Bd. 155, 65-70, 1956).

Es ist weiters bekannt, daß die nach den oben angeführten Verfahren hergestellten Immunglobulin-Präparate die Eigenschaft haben, Komplement unspezifisch zu fixieren (siehe dazu S. Baradun, Die Gammaglobulin-Therapie, Karger Verlag Basel, 1964) und daher lediglich für die intramuskuläre Applikation geeignet sind. Bei intravenöser Verabreichung können besonders bei Patienten mit Agammaglobulinämie heftige Reaktionen, die unter Umständen bis zum Schock führen, auftreten. Es wurde bereits nachgewiesen, daß aus natürlichem monomerem 7S-IgG-Immunglobulin im Verlaufe der Plasmafraktionierung teilweise dimere und oligomere Immunglobulinaggregate entstehen, die bei intravenöser Injektion das Komplementsystem aktivieren. Dieses Phänomen wird als antikomplementäre Aktivität bezeichnet und verursacht die oben angegebenen Reaktionen. Andererseits sind die in den meisten Krankheitsfällen therapeutisch erforderlichen Immunglobulin-Spiegel nur bei intravenöser Verabreichung zu erzielen, da intramuskulär injiziertes IgG infolge der schlechten Resorbierbarkeit und proteolytischer Abbaureaktionen an der Injektionsstelle nur teilweise und verzögert in den Blutkreislauf gelangt.

Zur Gewinnung von intravenös verträglichen Präparaten sind daher bereits verschiedene Verfahren entwickelt worden, die darauf abzielen, entweder die antikomplementären Eigenschaften nach der Fraktionierung zu vermindern oder das Entstehen von Immunglobulinaggregaten während eines herkömmlichen Fraktionierverfahrens zu verhindern.

Nach der von H.E. Schultze (Dtsch. med. Wochenschrift 87, 1643-1650, 1962) und später von A. Nisonoff (Science 132, 1770, 1970) vorgeschlagenen Methode gelingt es, IgG-Fraktionen durch Behandlung mit Pepsin von der antikomplementären Aktivität zu befreien. Durch die Enzymbehandlung des Immunglobulins, für die neben Pepsin auch Plasmin (S. Barandun et al., Vox. Sang. 28, 157, 1975) vorgeschlagen wurde, wird aber das Protein weitgehend in die einzelnen Antikörperfragmente, den $F(ab)_2$-Teil, den Fab-Teil und den Fc-Teil des Antikörpermoleküls, gespalten und es entstehen Präparate, die gegenüber natürlichem Immunglobulin eine deutlich verringerte Halbwertszeit und eingeschränkte biologische Wirksamkeit aufweisen (vgl. dazu die Arbeit von E. Merler et al., Vox Sang. 13, 102, 1967).

Aus der Arbeit von S. Barandun et al. (Vox Sang. 7, 157-174, 1962) ist bereits bekannt, daß Komplement inaktivierende Immunglobulinfraktionen durch eine Säurebehandlung bei einem pH-Wert von 3,8-4 ihre Antikomplementäraktivität vorübergehend verlieren. Die Säurebehandlung hat gegenüber dem enzymatischem Abbau den Vorteil, daß das IgG-Molekül weitgehend intakt bleibt und nicht modifiziert wird (J. Römer et al., Vox-Sang. 42, 62-73, 1982). Nach der bei Barandun angegebenen Arbeitsweise erhält man jedoch Präparate, die bei der Rückstellung des pH-Wertes in den Neutralitätsbereich als opalizierende Lösungen anfallen, aus denen bei längerem Stehen Niederschläge präzipitieren, und die während der Lagerung dazu neigen, wieder antikomplementär zu werden, sodaß ihre Verwendbarkeit für die intravenöse Injektion beschränkt ist.

Weiters ist aus der Arbeit von L.H. Frommhagen et al. in J. Immunol. 89, 336-343, 1962 bekannt, daß eine 20 minütige Wärmebehandlung des Human-Vollserums bei 63 °C geeignet ist, eventuell vorhandene Antikomplementäraktivität im nativen Serum zu vernichten. In der DE-PS-2 527 064 ist ein Verfahren zur Herstellung von nativem Immunglobulin für die intravenöse Applikation beschrieben, bei den das als Ausgangsmaterial dienende Humanplasma oder -serum für längere Zet auf 50-60 °C, vorzugsweise 2 Stunden lang auf 56 °C erhitzt wird, worauf die Gammaglobulinfraktion durch übliche Fraktionierverfahren, beispielsweise das Cohn'sche Alkoholfällungsverfahren oder die Rivanol-Ammonsulfat-Fraktionierung abgetrennt wird. Eine längere Erhitzung des Ausgangsmaterials hat aber den Nachteil, daß einzelne Plasmaproteine des Humanserums durch Denaturierung teilweise koagulieren und ausflocken können, wodurch die anschließe de Fraktionierung wesentlich erschwert und die Verwertbarkeit von weiteren Produkten der Plasmafraktionierung, eingeschränkt wird.

Die vorstehend angegebenen Methoden beschränken sich außerdem nur auf Anweisungen zur Herabsetzung der antikomplementären Aktivität von Immunglobulinpräparaten, die nach herkömmlichen Fraktionierverfahren, wie dem Cohn'schen Alkoholfällungsverfahren oder dem Rivanol-Ammonsulfat-Verfahren fraktioniert wurden. Die nach diesen Methoden gewonnenen Präparate sind jedoch nicht einheitlich und enthalten je nach Art des Ausgangsmaterials und der Fraktioniermethode neben der Hauptmenge an IgG-Immunglobulin noch Euglobuline sowie IgM- und IgA-Immunglobuline in unterschiedlichen Mengen. Die IgA-Fraktion besitzt zwar ebenfalls immunologische Abwehrstoffe gegen bestimmte Viren, kann aber bei Patienten mit Anti-IgA-Antikörpern heftige und unerwünschte Nebenwirkungen hervorrufen (vgl. dazu S. Barandun et al. Vox Song. 7, 157-174, 1962). Es ist daher für eine Immuntherapie von bakteriellen und viralen Infektionen mit IgG-Immunglobulin empfehlenswert, Präparate zu verwenden, bei denen der IgA-Gehalt soweit als möglich reduziert ist. In der DE-OS-2 404 265 ist ein verbessertes Verfahren zur Gewinnung von nach Klassen getrennten Immunglobulinkonzentraten beschrieben, bei dem die Euglobuline aus der Lösung einer mittels Alkoholfällung gewonnenen, rohen Immunglobulinfraktion durch Einstellen einer Leitfähigkeit von 5.0 mal $10^2$ bis 8.0 mal $10^3$ µS und eines pH-Werts von 4.5 bis 6, vorzugsweise von 5.1, ausgefällt und der Großteil des IgA durch Adsorption an Aluminiumhydroxid vom IgG abgetrennt werden kann, wobei zu 95 % reine Immunglobuline erhalten werden. In dieser DE-OS-2 404 265 ist jedoch keine Methode zur Herabsetzung der Antikomplementäraktivität beschrieben, sodaß diese Präparate ausdrücklich nur für die intramuskuläre Anwendung empfohlen werden.

Es wurde nun überraschenderweise gefunden, daß sich eine chemisch unmodifizierte, nebenwirkungsfreie IgG-Immunglobulinlösung für die intravenöse Anwendung nach einem verbesserten Verfahren herstellen läßt, bei dem die bisher mit der Entfernung der Antikomplementäraktivität verbundenen Nachteile vermieden werden und zu mehr als 95 % reine und lagerstabile IgG-Immunglobulinfraktionen mit einem IgA-Gehalt von weniger als 5 % gewonnen werden, wenn man eine unter ganz bestimmten Bedingungen durchgeführte Euglobulinfällung mit einer darauffolgenden adsorptiven Behandlung, einer daran anschließenden Säurebehandlung und Hitzebehandlung kombiniert, und aus der so erhaltenen Lösung durch mehrfache fraktionierte Fällungen zu Reaggregation neigende Proteine abtrennt und durch anschließende zweifache adsorptive Behandlung von niedermolekularen Begleitstoffen und proteolytischen Enzymen sowie pyrogenen Stoffen befreit.

Gegenstand der vorliegenden Erfindung ist demnach ein Verfahren zur Gewinnung einer chemisch unmodifizierten, nicht enzymatisch behandelten, nebenwirkungs- und rückstandsfreien, weitgehend reinen wässerigen IgG-Immunglobulinlösung für die intravenöse Applikation mit gegenüber natürlichem Plasma unveränderter Komplement bindender Aktivität hoher Stabilität und einem Gehalt an IgA-Immunglobulin von unter 5 Gew.% durch Reinigung und Anreicherung der IgG-Immunglobulinfraktion in mehreren Schritten, wie Fällen, Filtrieren, Adsorbieren und Dialysieren, welches dadurch gekennzeichnet ist, daß man

a) eine Fraktion II + III nach Cohn (Rohglobuline) oder eine durch das Rivanol-Ammonsulfat-Verfahren gewonnene rohe Immunglobulinfraktion in wässerige Lösung bringt, zur Entfernung der Fällungsmittel dialysiert und die Euglobuline durch Einstellen einer Leitfähigkeit von weniger als 3 mal $10^2$ µS und eines pH-Wertes von 5.3 bis 5.5 bei 0-10 °C ausfällt, aus dem Überstand die enzymatische Aktivität durch Adsorption an ein mineralisches Adsorbens abtrennt und im so gewonnenen Überstand den größten Teil der IgA-Fraktion des Immunglobulins durch Adsorption an 0.5-1.5 g/l DEAE (Diethylaminoethyl)-Ionenaustauscher bei einem pH-Wert von 6.5 bis 7.5 entfernt und das Adsorbens abtrennt, worauf man

b) die so gereinigte und angereicherte IgG-Immunglobulinlösung durch eine Säurebehandlung bei pH 2.5-4.2 stabilisiert und dann

c) die Lösung neutralisiert und bei annähernd neutralem pH-Wert 20-40 Minuten lang auf 40-50 °C erhitzt und nach dem Abkühlen den überwiegenden Teil des Ig-M, denaturierte Proteinfragmente oder andere Aggregate durch zweistufige fraktionierte Fällung mit jeweils an die Abtrennung des hochmolekularen Proteinniederschlages anschließender Ausfällung der den weiteren Reinigungsoperationen zugrundezulegenden IgG-Fraktion entfernt, die IgG-Fraktion der letzten Fällung in Wasser auflöst und dialysiert, worauf man

d) das Dialysat wieder in eine wässerige Lösung überfuhrt, diese zur Entfernung niedermolekularer Anteile mit Aktivkohle und die nach Abtrennung der Aktivkohle erhaltene Lösung zur Entfernung pyrogener Stoffe mit in situ gefälltem Aluminiumhydroxyd bei einem pH-Wert von 7.0 bis 8.0 behandelt, das Aluminiumhydroxyd abtrennt, aus der erhaltenen Lösung die IgG-Fraktion ausfällt und in die anwendungsfähige Lösung überführt.

Gegenstand der vorliegenden Erfindung sind ferner die nach diesem Verfahren hergestellten IgG-Immunglobilinlösungen, sowie die Präparate, die die genannten IgG-Immunglobulinlösungen enthalten.

Die Entfernung des Alkohols oder der Neutralsalze aus der als Ausgangsmaterial dienenden Fraktion II + III nach Cohn oder der nach dem Rivanol-Ammonsulfat-Verfahren erhaltenen rohen Immunglobulinfraktion erfolgt durch Suspendieren der pastenförmigen Plasmaproteine in demineralisiertem Wasser und anschließender dreitägiger Dialyse gegen demineralisiertes Wasser bei 4 °C.

Zur Ausfällung der Euglobuline wird in der DE-OS-2 404 265 die Einstellung einer Leitfähigkeit von 5.0 mal $10^2$ bis 8.0 mal $10^3$ µS und eines pH-Wertes von 4.5 bis 6, vorzugsweise eines pH-Wertes von 5.1, vorgeschlagen. Beim erfindungsgemäßen Verfahren wurde nun überraschenderweise festgestellt, daß eine optimale Fällung der Euglobuline ohne gleichzeitige Mitfällung des IgG dann erzielt werden kann, wenn man das Dialysat durch Zugabe der 10 bis 40-fachen Menge, vorteilhafterweise der 15-20-fachen Menge an demineralisiertem Wasser bezogen auf das Dialysatgewicht in Lösung bringt und durch Ansäuern mit verdünnten Säuren, vorzugsweise 20 %iger Essigsäure, einen pH-Wert von 5.3 bis 5.5, vorzugsweise 5.4, und eine Leitfähigkeit von weniger als 3 mal $10^2$ µS einstellt und die Fällung bei 0-10 °C, vorzugsweise bei 4 °C durchführt.

Nach der Abtrennung des Sediments, die auf übliche Weise durch Dekantieren, Filtrieren oder Zentrifugieren geschehen kann, wird dem euglobulinfreien Überstand zur Entfernung der enzymatischen Aktivität, insbesondere der proteolytischen Fermente, ein mineralisches Adsorbens, vorteilhafterweise Bariumsulfat, zweckmäßig in einer Menge von 20 bis 50 g/l, bevorzugt 30 g/l, zugesetzt und unter Rühren 2 bis 4 Stunden einwirken gelassen.

Nach Abtrennung des Adsorbens werden dem Überstand 0.5-1.5 g/l, vorteilhafterweise 1.0 g/l DEAE-Ionenaustauscher zugesetzt und der pH-Wert mit einer Base, vorteilhafterweise mit konzentriertem Ammoniak, auf 6.5-7.5, vorzugsweise 7.0, eingestellt und die Mischung 2 bis 4 Stunden gerührt, wobei der größte Teil des IgA an den DEAE-Ionenaustauscher adsorbiert wird.

Das Filtrat, welches die schon weitgehend gereinigte und angereicherte IgG-Immunglobulinlösung enthält, wird in Stufe b) zur Entfernung der antikomplementären Aktivität einer Säurebehandlung mit verdünnten Mineralsäuren unterzogen. Die Säurebehandlung kann in Abhängigkeit von der Dauer der Einwirkung bei einem pH-Wert von 2,5 bis 4,2, bevorzugt von 2,9 bis 3,9, erfolgen, wobei die Einstellung eines pH-Wertes von 3,3 bis 3,5 wiederum besonders bevorzugt ist. In einer günstigen Ausführungsform kann diese Behandlung in zwei Stufen durchgeführt werden, indem die Lösung zunächst mit 1N HCl bei 0-10 °C, bevorzugt bei 4 °C, auf einen pH-Wert von etwa 2.9 bis 3,1, gebracht und für 5 bis 10 Minuten bei diesem pH-Wert belassen wird. Anschließend wird durch Zugabe einer Base, bevorzugt von Ammoniak, ein pH-Wert von etwa 3.3 bis 3.5 eingestellt und die klare Lösung für 12-72 Stunden, bevorzugt 12 bis 24 Stunden bei 4 °C gehalten.

Durch die Säurebehandlung werden zwar die im Verlaufe der Plasmafraktionierung gebildeten dimeren und oligomeren Immunglobulinaggregate gespalten, die 7S-IgG-Komponente selbst bleibt bei dieser Behandlung aber überraschenderweise weitgehend intakt. Insbesondere wird durch die Säurebehandlung der Fc-Teil der Antikörpermoleküle, der für die spezifische Aktivierung des Komplementsystems und die Erhaltung der vollen prophylaktischen Wirksamkeit verantwortlich ist, im Gegensatz zur enzymatischen Behandlung nicht abgespalten (vgl. dazu J. Römer et al. Vox. Sang. 42, 74-80, 1982).

Die mit der Säurebehandlung verbundenen Vorteile bei der Entfernung der Antikomplementäraktivität wurden aber bei den bisher bekannten Verfahren durch die oben angeführten Nachteile, wie Entstehen von opalisierenden Lösungen bei Rückstellung des pH-Wertes in den Neutralitätsbereich, Bildung von Niederschlägen und Wiederauftreten der Antikomplementäraktivität während der Lagerung aufgehoben. Es ist daher ein überraschendes Ergebnis, daß diese Nachteile durch technisch sehr einfach durchzuführende und wirkungsvolle Maßnahmen in Stufe c) des erfindungsgemäßen Verfahrens beseitigt werden können. Durch Erhitzen der auf einen pH-Wert von 6.5 bis 7.5, vorzugsweise 7.0, eingestellten Lösung auf 40 °C bis 50 °C für 20 bis 40 Minuten, bevorzugt auf 42 °C bis 45 °C für 30 Minuten, werden in der Lösung vorhandene Proteinfragmente denaturiert und können zusammen mit dem Großteil des IgM und anderen Aggregaten durch fraktionierte Fällung aus der Lösung entfernt werden. Das Erhitzen kann auf beliebige Art geschehen, wird aber bevorzugt durch Einleiten von Dampf in die gerührte Lösung durchgeführt.

Unmittelbar nach der Erhitzungsperiode wird die Lösung auf 20 °C bis 25 °C abgekühlt und die Proteine mit einem höherem Molekulargewicht als IgG, wie IgM, denaturierte Proteinfragmente oder andere noch vorhandene Aggregate durch Zugabe eines Neutralsalzes oder niederen Alkohols, vorzugsweise durch Zugabe von Ammonsulfat bis zu einer Konzentration von 150 g/l, ausgefällt. Die schonende und kurzzeitige Hitzebehandlung beim erfindungsgemäßen Verfahren hat den Vorteil, daß einerseits das gereinigte und angereicherte 7S-IgG-Immunglobulin weder denaturiert noch aggregiert, daß es aber andererseits gelingt, ein Wiederauftreten der Antikomplementäraktivität nach der Säurebehandlung in der gereinigten IgG-Fraktion unter wesentlich milderen Bedingungen zu verhindern, als es beim Erhitzen des unfraktionierten Humanplasmas oder -serums der Fall wäre.

Aus dem Filtrat der obigen Fällung wird die IgG-Fraktion durch Erhöhung der Salzkonzentration oder weitere Alkoholzugabe, vorzugsweise durch Zugabe von Ammonsulfat bis zu einer Konzentration von 300 g/l, ausgefällt. Die durch

Zentrifugation oder Filtration abgetrennte IgG-Fällung wird zur Gewinnung eines besonders reinen Produkts noch einmal in Lösung gebracht und durch fraktionierte Fällung bei pH 4.0 bis 4.5, vorzugsweise bei pH 4.2, mit einem Neutralsalz, vorzugsweise mit Ammonsulfat, bis zu einer Konzentration von 100 bis 160 g/l, vorzugsweise etwa 120 g/l, von bisher noch nicht abgetrennten Aggregaten und Proteinen mit einem höherem Molekulargewicht als IgG-Immunglobulin befreit. Im neutralisierten Filtrat wird die reine IgG-Fraktion schließlich durch Erhöhung der Salzkonzentration oder weitere Alkoholzugabe, vorzugsweise durch Zugabe von Ammonsulfat bis zu einer Konzentration von 300 g/l, ausgefällt, der Niederschlag abgetrennt und zur Befreiung von Fällungsmittel gegen gekühltes Wasser dialysiert.

Die Stabilität und Freiheit von pyrogenen Stoffen kann beim erfindungsgemäßen Verfahren überraschenderweise durch die abschließende Behandlung der IgG-Immunglobulinlösungen mit Aktivkohle und Aluminiumhydroxid erzielt werden. Man geht vorteilhafterweise so vor, daß man das Dialysat aus Stufe c) mit demineralisiertem Wasser zu einer 1 bis 2 Gew.% Proteine enthaltenden Lösung verdünnt, diese mit verdünnter Essigsäure auf pH 4.5-5.0, vorzugsweise auf einem pH von etwa 4.8, einstellt, mit 0.2 g Aktivkohle/g Protein versetzt und anschließend 2 Stunden rührt. Die Behandlung mit Aktivkohle hat den Vorteil, daß eine Lösung hergestellt werden kann, die von niedermolekularen Begleitstoffen und proteolytischen Enzymen völlig frei ist, wodurch die erfindungsgemäßen Lösungen außerordentlich lagerstabil werden. Nach der Abtrennung der Aktivkohle, vorteilhafterweise durch Zentrifugieren, wird die erhaltene Lösung mit einer Base, bevorzugt konz. Ammoniak, auf einen pHWert von 7.0 bis 7.5 eingestellt und auf 37 °C erwärmt. Die in situ-Fällung von Aluminiumhydroxyd kann dann durch Zugabe von Al-Salzen zur Lösung, beispielsweise von Aluminiumalaun, vorzugsweise in einer Menge von 0.2 g/l Protein und anschließende Einstellung eines leicht basischen pH-Wertes von etwa 7.0 bis 8.0 erfolgen. Das ausfallende Aluminiumhydroxid adsorbiert unter diesen Bedingungen pyrogene Verunreinigungen, während das IgG-Immunglobulin im Überstand bleibt.

Aus dem Filtrat schließlich wird die reine IgG-Fraktion zweckmäßigerweise durch Zugabe eines Neutralsalzes oder eines niederen Alkohols, vorzugsweise von Ammonsulfat bis zu einer Konzentration von etwa 300 g/l, ausgefällt. Das erfindungsgemäß hergestellte IgG-Immunglobulin wird in der für Immunglobuline bekannten Weise, beispielsweise in einer etwa 1.5 % Glycin und etwa 0.7 % Natriumchlorid enthaltenden Lösung aufgelöst, steril filtriert und als stabile, wässerige Lösung mit einem Proteingehalt von beispielsweise etwa 5 Gew.% in Ampullen für die intravenöse Applikation zur Verfügung gestellt.

Die Reinheit und Ausbeute der erhaltenen Produkte wird mit gelchromatographischen Methoden bestimmt. Die erfindungsgemäß hergestellten IgG-Präparationen enthalten nach diesen Kriterien zu mehr als 95 % reines IgG-Immunglobulin und weniger als 5 % igA-Immunglobulin bezogen auf das Gesamtprotein. Der IgG-Anteil besteht zu mehr als 90 % aus der 7S-Komponente und enthält weniger als 10 % Aggregate.

Die Ausbeute an der reinen IgG-Fraktion beträgt 20-30 Liter aus 1 000 l humanen Blutplasma. Die erfindungsgemäß hergestellten wässerigen i.v. IgG-Immunglobulinpräparate können über Monate hinweg in unverändertem Zustand gelagert werden und sind für die intravenöse Applikation an Menschen als spezifische Immunprophylaktika und -therapeutika sehr gut geeignet.

Das folgende Beispiel erläutert die Erfindung näher ohne sie zu beschränken :

Beispiel

56,3 g COHN II + III Präzipitat (Rohglobuline) werden in 100 ml demineralisiertem (d. m.) Wasser gelöst und 72 Stunden gegen fließendes demineralisiertes Wasser bei + 4 °C dialysiert. Durch Zugabe von 2 700 ml d. m. Wasser wird eine Leitfähigkeit von 1.9 mal $10^2 \mu S$ erreicht und anschließend ein pH-Wert von 5,35 mit Essigsäure eingestellt. Das entstandene Präzipitat wir durch 24-stündige Sedimentation abgetrennt. Der Überstand wird mit 30 g/l Bariumsulfat versetzt, 2 Stunden gerührt und das Bariumsulfat anschließend durch Zentrifugieren abgetrennt. Das Zentrifugat wird durch Zugabe von Ammoniak konz. auf pH 7,18 eingestellt, mit 1.0 g/l DEAE-Sephadex A-50$^R$ versetzt, 3 Stunden gerührt und das beladene DEAE-Sephadex ebenfalls abzentrifugiert. Durch Zugaben von In Salzsäure wird der pH-Wert im Überstand auf 3.08 eingestellt und nach 5 Minuten mit Ammoniak konz. auf 3,45 korrigiert, die Lösung bleibt 48 Stunden bei + 4 °C stehen. Der pH-Wert wird durch weitere Zugabe von Ammoniak konz. auf 7.2 eingestellt, die Lösung auf 42 °C erhitzt und 40 Minuten bei dieser Temperatur gehalten. Anschließend wird auf 20 °C abgekühlt. Durch Zugabe von 150 g/l Ammonsulfat werden hochmolekulare (IgM-Immunglobuline) und denaturierte Anteile aus der Lösung ausgefällt, durch Filtration abgetrennt und verworfen. Aus dem Filtrat wird durch Zugabe von weiteren 150 g/l Ammonsulfat das bereits angereicherte IgG-Immunglobulin präzipitiert und durch Filtration isoliert.

Das gewonnene Präzipitat wird gelöst, mit In Salzsäure auf pH 4,25 eingestellt und mit 130 g/l Ammonsulfat versetzt. Der entstandene Niederschlag wird durch Filtration entfernt und verworfen. das Filtrat wird neutralisiert (pH 7.10), mit weiteren 170 g/l Ammonsulfat versetzt und das weitgehend aggregatfreie IgG-Globulin präzipitiert. Die erhaltenen 9.9 g Präzipitat (enthaltend 1,8 g Protein) werden auf 1.5 Gew.% Protein gelöst, der pH-Wert mit Essigsäure auf 4,8 eingestellt, mit 0.2 g Aktivkohle pro Gramm Protein versetzt und 2 Stunden gerührt. Anschließend wird die Aktivkohle durch Zentrifugation abgetrennt.

Das Zentrifugat wird mit Ammoniak konz. auf pH 7.2 neutralisiert, auf 37 °C erwärmt, mit 0,2 g Ammoniumalaun pro Gramm Protein versetzt, worauf durch weitere Zugabe von Ammoniak konz. bis zum pH-Wert von 7.8 Aluminiumhydroxid in situ ausgefällt wird. Nach 1-stündigem Rühren wird das Aluminiumhydroxid abfiltriert, das Immunglobulin durch Präzipitation mit 300 g/l Ammoniumsulfat aus dem Filtrat ausgefällt, und durch Filtration isoliert.

Das gewonnene Präzipitat wird 72 Stunden gegen d.m. Wasser dialysiert, auf 5 Gew.% Protein, 1.5 Gew.% Glycin und 0.7 Gew.% Natriumchlorid eingestellt, der pH-Wert mit Salzsäure auf 6,9 korrigiert und anschließend sterilfiltriert.

Die Ausbeute beträgt 24 ml intravenös applizierbares Immunglobulin.

**Patentansprüche**

1. Verfahren zur Gewinnung einer chemisch unmodifizierten, nicht enzymatisch behandelten, nebenwirkungs- und rückstandsfreien, weitgehend reinen wässerigen IgG-Immunglobulinlösung für die intravenöse Applikation mit gegenüber natürlichem Plasma unveränderter Antikomplementäraktivität, hoher Stabilität und einem Gehalt an IgA-Immunglobulin von unter 5 Gew.% durch Reinigung und Anreicherung der IgG-Immunglobulinfraktion in mehreren Schritten, wie Fällen, Filtrieren, Adsorbieren und Dialysieren, dadurch gekennzeichnet, daß man

a) eine Fraktion II + III nach Cohn (Rohglobulin) oder eine durch das Rivanol-Ammonsulfat-Verfahren gewonnene rohe Immunglobulinfraktion in wässerige Lösung bringt, zur Entfernung der Fällungsmittel dialysiert und die Euglobuline durch Einstellen einer Leitfähigkeit von weniger als 3 mal $10^2$ µS und eines pH-Wertes von 5.3 bis 5.5 bei 0-10 °C ausfällt, aus dem Überstand die enzymatische Aktivität durch Adsorption an ein mineralisches Adsorbens abtrennt und im so gewonnenen Überstand den größten Teil der IgA-Fraktion des Immunglobulins durch Adsorption an 0.5-1.5 g/l Diethylaminoethyl (DEAE) lonenaustauscher bei einem pH-Wert von 6.5 bis 7.5 entfernt und das Adsorbens abtrennt, worauf man

b) die so gereinigte und angereicherte IgG-Immunglobulinlösung durch eine Säurebehandlung bei pH 2.5-4.2 stabilisiert, und dann

c) die Lösung neutralisiert und bei neutralem pH-Wert 20-40 Minuten lang auf 40-50 °C erhitzt und nach dem Abkühlen den überwiegenden Teil des IgM, denaturierte oder andere Aggregate durch zweistufige fraktionierte Fällung mit jeweils an die Abtrennung des hochmolekularen Proteinniederschlages anschließender Ausfällung der den weiteren Reinigungsoperationen zugrundezulegenden IgG-Fraktion entfernt, die IgG-Fraktion der letzten Fällung in Wasser auflöst und dialysiert, worauf man

d) das Dialysat wieder in eine wässerige Lösung überführt, diese zur Entfernung niedermolekularer Anteile mit Aktivkohle und die nach Abtrennung der Aktivkohle erhaltene Lösung zur Entfernung pyrogener Stoffe mit in situ gefälltem Aluminiumhydroxyd bei einem pH-Wert von 7.0 bis 8.0 behandelt, das Aluminiumhydroxyd abtrennt, aus der erhaltenen Lösung die IgG-Fraktion ausfällt und in die anwendungsfähige Lösung überführt.

2. Verfahren nach Anspruch 1, dadurch gekennzeichnet, daß man in Stufe a) die Euglobuline aus der wässerigen Lösung der rohen Immunglobulinfraktion bei einem pH-Wert von 5.4 und bei einer Temperatur von etwa 4 °C ausfällt.

3. Verfahren nach den Ansprüchen 1 und 2, dadurch gekennzeichnet, daß man in Stufe a) als mineralisches Adsorbens Bariumsulfat verwendet und eine Menge von 20-50 g/l einsetzt.

4. Verfahren nach den Ansprüchen 1 bis 3, dadurch gekennzeichnet, daß in Stufe a) der DEAE-Ionenaustauscher in einer Menge von etwa 1.0 g/l zugesetzt und ein pH-Wert von etwa 7.0 eingestellt wird.

5. Verfahren nach den Ansprüchen 1 bis 4, dadurch gekennzeichnet, daß die Säurebehandlung in Stufe b) bei einem pH-Wert von 3.3 bis 3.5 durchgeführt wird.

6. Verfahren nach den Ansprüchen 1 bis 4, dadurch gekennzeichnet, daß die Säurebehandlung in Stufe b) in zwei Schritten durchgeführt wird, indem durch Ansäuern mit IN HCl bei 4 °C für 5 bis 10 Minuten ein pH-Wert von etwa 2.9 bis 3.1 eingestellt und anschließend nach Rückstellung des pH-Wertes auf etwa 3.3 bis 3.5 mit Ammoniak für 12 bis 24 Stunden inkubiert wird.

7. Verfahren nach den Ansprüchen 1 bis 6, dadurch gekennzeichnet, daß das Erhitzen der Lösung in Stufe c) bei einem pH-Wert von 7.0 vorgenommen wird, wobei für etwa 30 Minuten eine Temperatur von 42 °C bis 45 °C eingehalten wird.

8. Verfahren nach den Ansprüchen 1 bis 7, dadurch gekennzeichnet, daß die erste Stufe der fraktionierten Fällungen in Stufe c) durch Zugabe von Ammonsulfat bis zu einer Konzentration von etwa 150 g/l durchgeführt wird, worauf die IgG-Fraktion nach Abtrennung des Präzipitats im Überstand durch Zugabe von Ammonsulfat bis zu einer Gesamtkonzentration von etwa 300 g/l ausgefällt, abgetrennt und wieder in Lösung gebracht wird.

9. Verfahren nach den Ansprüchen 1 bis 8, dadurch gekennzeichnet, daß die zweite Stufe der fraktionierten Reinigungsfällungen in Stufe c) bei einem pH-Wert von 4.0 bis 4.5 durch Zugabe von Ammonsulfat bis zu einer Konzentration von 100 bis 160 g/l durchgeführt wird, worauf die IgG-Fraktion nach Abtrennung des Präzipitats im Überstand durch Zugabe von Ammonsulfat bis zu einer Gesamtkonzentration von etwa 300 g/l ausgefällt wird.

10. Verfahren nach den Ansprüchen 1 bis 9, dadurch gekennzeichnet, daß die IgG-Immunglobulinfraktion in Stufe d) zu einer Lösung mit einem Proteingehalt von 1 bis 2 Gew.% aufgelöst und die Aktivkohlebehandlung mit einer Aktivkohlemenge von etwa 0.2 g pro g Protein bei einem pH-Wert von etwa 4.8 durchgeführt wird.

11. Verfahren nach den Ansprüchen 1 bis 10, dadurch gekennzeichnet, daß man die Aluminiumhydroxydbehandlung in Stufe d) durch Versetzen der von der Aktivkohle befreiten Lösung mit 0.2 g Aluminiumalaun/g Protein bei pH 7.0 und 37 °C und anschließende Fällung des Aluminiumhydroxyds durch Erhöhen des pH-Wertes auf 7.5 bis 8.0 durchführt.

12. Verfahren nach den Ansprüchen 1 bis 11, dadurch gekennzeichnet, daß durch Auflösen der reinen IgG-Fraktion in einer etwa 1,5 % Glycin und etwa 0,7 % Natriumchlorid enthaltenden wässerigen Lösung und Sterilfiltration eine etwa 5 %ige proteinhältige stabile Lösung für die intravenöse Applikation hergestellt wird.

13. IgG-Immunglobulinlösung für die intravenöse Applikation, hergestellt nach dem Verfahren gemäß den Ansprüchen 1 bis 12.

14. Immunglobulinpräparate, enthaltend eine IgG-Immunglobulinlösung gemäß Anspruch 13.

**Claims**

1. Process for obtaining a chemically unmodified, non-enzymatically treated, essentially pure aqueous IgG immunoglobulin solution which is free of side effects and residues, is for intravenous administration, and has anticomplementary activity which is no different from that of natural plasma, high stability and a content of less than 5 % by weight of IgA immunoglobulin, by purification and enrichment of the IgG immunoglobulin fraction in several steps, such as precipitation, filtration, adsorption and dialysis, characterized in that

a) a Cohn fraction II + III (crude globulin) or a crude immunoglobulin fraction obtained by the Rivanol/ammonium sulphate process is converted into an aqueous solution, which is dialysed to remove the precipitant, and the euglobulins are precipitated by adjusting to a conductivity of less than $3 \times 10^2$ µS and a pH of 5.3 to 5.5 at 0-10 °C, the enzymatic activity is removed from the supernatant by adsorption onto a mineral adsorbent, and most of the IgA fraction of the immunoglobulin in the supernatant which has thus been obtained is removed by adsorption onto 0.5-1.5 g/l diethylaminoethyl-ion exchanger at a pH of 6.5 to 7.5, and the adsorbent is removed, whereupon

b) the IgG immunoglobulin solution which has thus been purified and enriched is stabilized by acid treatment at pH 2.5-4.2, and then

c) the solution is neutralized and heated at 40-50 °C and neutral pH for 20-40 minutes and, after cooling, the major part of the IgM, denatured or other aggregates are removed by two-stage fractional precipitation, with each removal of the high molecular weight protein precipitate being followed by precipitation of the IgG fraction to be used as a basis for the further purification operations, and the IgG fraction from the final precipitation is dissolved in water and dialysed, whereupon

d) the dialysate is once more converted into an aqueous solution, the latter is treated with active charcoal to remove low molecular weight fractions, and the solution obtained after removal of the active charcoal is treated with aluminium hydroxide, which has been precipitated in situ, at a pH of 7.0 to 8.0 to remove pyrogenic substances, the aluminium hydroxide is removed, and the IgG fraction is precipitated from the resulting solution and converted into the solution which can be used.

2. Process according to Claim 1, characterized in that, in stage a), the euglobulins are precipitated from the aqueous solution of the crude immunoglobulin fraction at a pH of 5.4 and at a temperature of about 4 °C.

3. Process according to Claims 1 and 2, characterized in that, in stage a), barium sulphate is used as mineral adsorbent, and an amount of 20-50 g/l is used.

4. Process according to Claims 1 to 3, characterized in that, in stage a), the DEAE-ion exchanger is added in an amount of about 1.0 g/l, and a pH of about 7.0 is set up.

5. Process according to Claims 1 to 4, characterized in that the acid treatment in stage b) is carried out at a pH of 3.3 to 3.5.

6. Process according to Claims 1 to 4, characterized in that the acid treatment in stage b) is carried out in two steps, comprising a pH of about 2.9 to 3.1 being set up by acidification with 1N HCl at 4 °C for 5 to 10 minutes, and then, after resetting the pH to about 3.3 to 3.5 with ammonia, incubation is carried out for 12 to 24 hours.

7. Process according to Claims 1 to 6, characterized in that the heating of the solution in stage c) is carried out at a pH of 7.0, the temperature being maintained at 42 °C to 45 °C for about 30 minutes.

8. Process according to Claims 1 to 7, characterized in that the first stage of the fractional precipitations in stage c) is carried out by addition of ammonium sulphate to a concentration of about 150 g/l, whereupon, after removal of the precipitate, the IgG fraction in the supernatant is precipitated by addition of ammonium sulphate to a total concentration of about 300 g/l, is removed and again converted into a solution.

9. Process according to Claims 1 to 8, characterized in that the second stage of the fractional purifying precipitations in stage c) is carried out at a pH of 4.0 to 4.5 by addition of ammonium sulphate to a concentration of 100 to 150 g/l, whereupon, after removal of the precipitate, the IgG fraction in the supernatant is precipitated by addition of ammonium sulphate to a total concentration of about 300 g/l.

10. Process according to Claims 1 to 9, characterized in that the IgG immunoglobulin fraction in stage d) is dissolved to give a solution with a protein content of 1 to 2 % by weight, and the active charcoal treatment is carried out with an amount of active charcoal of about 0.2 g per g of protein at a pH of about 4.8.

11. Process according to Claims 1 to 10, characterized in that the aluminium hydroxide treatment in stage d) is carried out by mixing the solution, from which active charcoal has been removed, with 0.2 g of aluminium alum per g of protein at pH 7.0 and 37 °C, and subsequently precipitating the aluminium hydroxide by increasing the pH to 7.5 to 8.0.

12. Process according to Claims 1 to 11, characterized in that a stable solution which contains about 5 % protein and is intended for intravenous administration is prepared by dissolving the pure IgG fraction in an aqueous solution which contains about 1.5 % glycine and about 0.7 % sodium chloride, and sterilizing by filtration.

13. IgG immunoglobulin solution for intravenous administration, prepared by the process according to Claims 1 to 12.

14. Immunoglobulin products containing an IgG immunoglobulin solution according to Claim 13.

## Revendications

1. Procédé pour la préparation d'une solution aqueuse d'immunoglobuline IgG de pureté poussée, pour l'administration intraveineuse, non modifiée chimiquement, non traitée enzymatiquement, dépourvue d'effets secondaires et de résidus, présentant une activité complémentaire inchangée vis-à-vis du plasma naturel, une stabilité élevée et une teneur en immunoglobuline IgA inférieure à 5 % en poids, par purification et concentration de la fraction d'immunoglobuline IgG en plusieurs stades, tels que précipitation, filtration, adsorption et dialyse, caractérisé en ce que

a) On dialyse une fraction II + III selon Cohn (globulinesbrutes) ou on amène en solution aqueuse une fraction d'immunoglobuline brute récupérée par le procédé de Rivanol au sulfate d'ammonium, pour l'élimination de l'agent de précipitation et on précipite les euglobulines par établissement d'une conductivité inférieure à 3 fois $10^2$ µS et d'une valeur de pH de 5,3 à 5,5 à 0-10 °C, on élimine l'activité enzymatique à partir du surnageant par adsorption sur un adsorbant minéral, on élimine dans le surnageant ainsi récupéré la plus grande partie de la fraction IgA de l'immunoglobuline par adsorption sur 0,5-1,5 g/l sur d'une résine échangeuse d'ions-DEAE (diéthylaminoéthyl) à une valeur de pH de 6,5 à 7,5 et on sépare l'adsorbant, après quoi

b) on stabilise la solution d'immunoglobuline IgG, ainsi purifiée et concentrée, par un traitement acide à pH 2,5-4,2, puis

c) on neutralise la solution, on la chauffe à valeur de pH neutre pendant 20-40 minutes à 40-50 °C et, après le refroidissement, on élimine la partie principale de l'IgM et des agrégats dénaturés ou autres par précipitation fractionnée en deux étapes avec pour chacun d'eux et consécutivement à la séparation du dépôt protéinique à poids moléculaire élevé, une précipitation de la fraction d'IgG destinée à être soumise aux opérations de purification ultérieures, on dissout dans l'eau la fraction d'IgG de la dernière précipitation, après quoi

d) on convertit de nouveau le dialysat en une solution aqueuse, on traite celle-ci avec du charbon actif pour l'élimination des fractions à bas poids moléculaires, tandis qu'après séparation du charbon actif, on traite la solution obtenue pour l'élimination des substances pyrogènes avec de l'hydroxyde d'aluminium précité in situ à une valeur de pH de 7,0 à 8,0, on sépare l'hydroxyde d'aluminium, on précipite la fraction d'IgG à partir de la solution obtenue et on convertit ladite fraction en solution utilisable.

2. Procédé selon la Revendication 1, caractérisé en ce que dans l'étape a), on précipite les euglobulines à partir de la solution aqueuse de la fraction d'immunoglobuline brute à une valeur de pH de 5,4 et à une température d'environ 4 °C.

3. Procédé selon les Revendications 1 et 2, caractérisé en ce que dans l'étape a), on utilise comme adsorbant minéral du sulfate de baryum que l'on introduit en une quantité de 20-50 g/l.

4. Procédé selon les Revendications 1 à 3, caractérisé en ce que dans le Stade a), on ajoute la résine échangeuse d'ions — DEAE en une quantité d'environ 1,0 g/l et on établit une valeur de pH d'environ 7,0.

5. Procédé selon les Revendications 1 à 4, caractérisé en ce que l'on effectue le traitement acide dans l'étape b) à une valeur de pH de 3,3 à 3,5.

6. Procédé selon les Revendications 1 à 5, caractérisé en ce que l'on effectue le traitement acide dans l'étape b) en deux temps, consistant en ce que par acidification avec HCl 1N à 4 °C pendant 5 à 10 minutes, on établit une valeur de pH d'environ 2,9 à 3,1 et consécutivement, après avoir ramené la valeur du pH à environ 3,3 à 3,5 avec de l'ammoniaque, on fait incuber pendant 12 à 24 heures.

7. Procédé selon les Revendications 1 à 6, caractérisé en ce que l'on procède au chauffage de la solution dans l'étape c) à une valeur de pH de 7,0 en maintenant pendant environ 30 minutes une température de 42 °C à 45 °C.

8. Procédé selon les Revendications 1 à 7, caractérisé en ce que l'on effectue la première étape des précipitations fractionnées dans l'étape c) par addition de sulfate d'ammonium jusqu'à une concentration d'environ 150 g/l, après quoi on précipite la fraction d'IgG après séparation du précipité dans le surnageant par addition de sulfate d'ammonium jusqu'à une concentration totale d'environ 300 g/l, on sépare ladite fraction et on l'amène de nouveau en solution.

9. Procédé selon les Revendications 1 à 8, caractérisé en ce que l'on effectue la deuxième étape des précipitations fractionnées de purification dans l'étape c) à une valeur de pH de 4,0 à 4,5 par addition de sulfate d'ammonium jusqu'à une concentration de 100 à 160 g/l, après quoi on précipite la fraction d'IgG après séparation du précipité dans le surnageant par addition de sulfate d'ammonium jusqu'à une concentration totale d'environ 300 g/l.

10. Procédé selon les Revendications 1 à 9, caractérisé en ce que l'on dissout la fraction d'immunoglobuline IgG dans l'étape d) de manière à obtenir une solution ayant une teneur en protéines de 1 à 2 % en poids et on effectue le traitement au charbon actif avec une quantité de charbon actif d'environ 0,2 g par gramme de protéine à une valeur de pH d'environ 4,8.

11. Procédé selon les Revendications 1 à 10, caractérisé en ce que l'on effectue le traitement par l'hydroxyde d'aluminium dans l'étape d) en additionnant la solution libérée du charbon actif avec 0,2 g d'alun d'aluminium/g de protéine à pH 7,0 et 37 °C et en procédant à une précipitation consécutive de l'hydroxyde d'aluminium par élévation de la valeur du pH à 7,5-8,0.

12. Procédé selon les Revendications 1 à 11, caractérisé en ce que par dissolution de la fraction d'IgG pure dans une solution aqueuse contenant environ 1,5% de glycine et environ 0,7 % de chlorure de sodium et filtration stérile, on prépare une solution stable à environ 5 % renfermant des protéines pour l'administration intraveineuse.

13. Solution d'immunoglobuline IgG destinée à l'administration intraveineuse, préparée suivant le procédé selon les Revendications 1 à 12.

14. Préparations d'immunoglobuline contenant une solution d'immunoglobuline IgG selon la Revendication 13.